# EUROPEAN PATENT SPECIFICATION

(11) **EP 3 115 016 B1**
(45) Date of publication and mention of the grant of the patent: **27.02.2019**
(21) Application number: 16175532.7
(22) Date of filing: 05.01.2007
(51) Int. Cl.: A61C 1/08, A61C 1/14, A61B 90/00, A61C 5/44, A61C 1/12

(54) **DENTAL HANDPIECE**
DENTALES HANDSTÜCK
PIECE A MAIN DENTAIRE

(30) Priority: 06.01.2006 GB 0600222; 10.01.2006 GB 0600359
(43) Date of publication of application: 11.01.2017
(62) Divisional of application: 07702594.8
(73) Proprietor: Dentsply Implants NV, 3500 Hasselt (BE)
(72) Inventor: ESPOSTI, Alessio, B-1170 Bruxelles (BE); VAN LIERDE, Carl, B-9402 Meerbeke (BE)
(74) Representative: IPLodge bvba

(56) References cited:
- EP-A2- 0 328 911
- US-A- 4 217 098
- US-A1- 2002 025 504
- US-A1- 2002 137 003
- US-B1- 6 575 747

## Description

### FIELD OF THE INVENTION

This invention relates to a dental handpiece and a process of fitting dental implants using the handpiece.

### BACKGROUND TO THE INVENTION

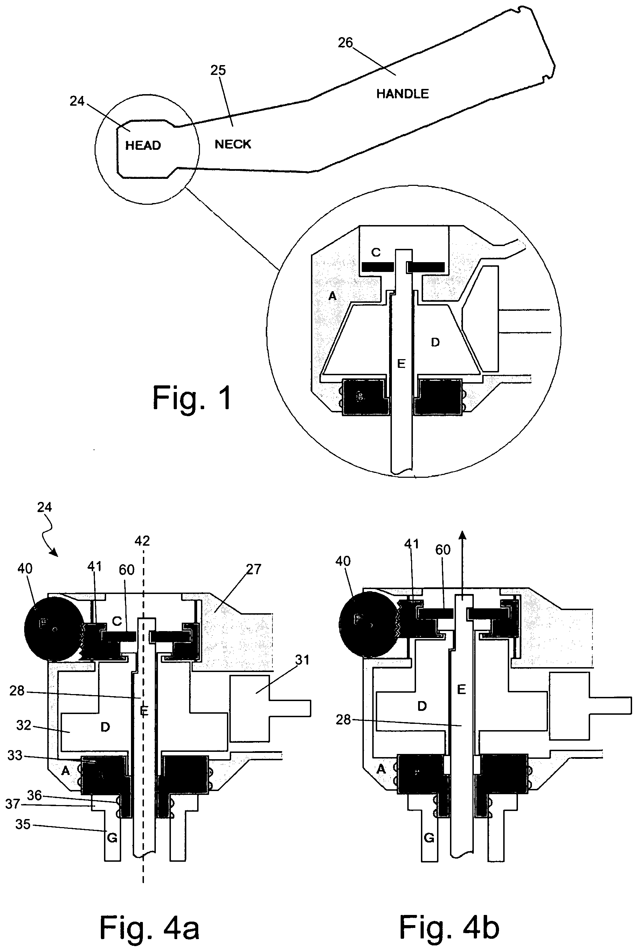

In dental treatment drive apparatuses for rotary tools, known as handpieces, are well known. Dental handpieces come in several shapes and most commonly are either straight or have a swan neck. Figure 1 shows a typical swan-necked dental handpiece, which comprises a handle 26, a neck 25 and a head 24 in which a rotary tool (e.g. drill, bur, implant driver) is fixed. The handpiece is typically driven by a drive shaft or belt, which extends between the head of the handpiece and a remotely located drive motor. The drive shaft transmits the rotational movement of the motor to the handpiece. Handpieces can alternatively be driven by electric or pneumatic drive motors. Handpieces are used to drive tools during various dentistry operations, such as filling cavities, cleaning and the fitting of dental implants.

A known dentistry operation is the fitting of artificial teeth by the use of dental implants. Implants are fixed in the jawbone of a patient. A dental superstructure is fitted on the implants and the superstructure supports artificial teeth of the required size and shape. A custom-made surgical template can be used as part of the implant process. The template has a set of bore holes which define the positions where holes need to be drilled and implants fitted. The template guides the surgical tools which are used when preparing implant cavities and inserting the implants. These templates fit on a defined mating region in the mouth and feature bore tubes. The templates can be designed based on an implant plan defined on computer using planning software such as SimPlant™.

In order to optimise not only the entry point positioning and inclination of the implants but also their insertion depth, dedicated components (e.g. drills, bushings and fixture mounts) have been developed. International Patent Application WO2004/075771, US Patent Application US2005/0170311 and US 4,998,881 describe a process of placing implants using a template. Drill bushings are inserted into bore tubes in the template. Drilling is performed using specially designed drills through these bushings. Small tolerances between drill and bushing limit the possible deviations from the optimal drill path and a flange on the drill acts as a physical stop to control the depth of penetration into the jaw bone. After drilling, the drill bushings are removed from the template and the implants are placed through the bore tubes. A tool can then be used to apply torque to drive the implant into the jawbone. One of the disadvantages of this method relates to the removal of the drill bushings. Due to limited space in the mouth of the patient, manipulating the drill bushings is difficult. Typically, once holes have been drilled in the jaw, the surgical template must be removed from its position in the patient's mouth to take out the drill bushings. The template is then repositioned.

Document US 6,575,747 B1 discloses a dental handpiece comprising: a housing having an inlet through which a shank of a drill bit can be inserted. This known handpiece further comprises a tool retaining mechanism for releasably retaining the shank of the rotary dental tool, the mechanism retaining the tool such that the tool extends from the housing, through the inlet, along a first axis. This handpiece further comprises an adjustment mechanism arranged to adjust the position of the tool relative to a template to be placed over the gums of the patient in the direction of the first axis, to thereby vary the depth of travel of the tool in the template.

While the aforementioned tools can be used in combination with a handpiece, their use is limited by the extent that they are compatible with existing implant systems. One of the disadvantages of the above methods is that dedicated components are required per implant brand and implant line (internal or external connection). The need for an extensive set of tools and accessories of bespoke sizes makes the solution expensive and inflexible.

### SUMMARY OF THE INVENTION

An object of the present invention is to provide an improved dental handpiece and a process of fitting dental implants using the handpiece. An advantage of the present invention is that it can overcome at least one of the known problems with prior art devices.

A first aspect of the present invention provides a dental handpiece comprising: a housing having an inlet through which a shank of a rotary dental tool can be inserted; a tool retaining mechanism for releasably retaining the shank of the rotary dental tool, the mechanism retaining the tool such that the tool extends from the housing, through the inlet, along a first axis; and an adjustment mechanism arranged to act on the tool retaining mechanism to adjust the position of the tool retaining mechanism within the housing, in the direction of the first axis, to thereby vary the length of tool which projects from the housing. The adjustment may be manual; motorised or automatic. The adjustment may be in function of an output of a computer planning. The adjustment mechanism provides a simple way for a user to vary the working depth of the tool. As an example, a set of implant sites may require holes to be drilled of different depths. The adjustment mechanism can be operated before each drilling operation to vary the drill to the required length for that implant site.

Preferably, the dental handpiece in accordance with this aspect of the invention can be used in combination with a guide member as previously recited, which has a flange that defines a working depth for the tool.

A drive can be provided for rotating a retained tool about the first axis. The drive can be any of a pneumatic, hydraulic or electrical drive or components for a manual drive.

A dental handpiece according to any of the embodiments of the present invention can be included as part of a dental apparatus that also includes a template having at least one bore hole which defines a position at which a rotary tool is required to be used, the bore hole having a diameter which is substantially equal to the outer diameter of the guide member.

A tubular guide member can be mounted coaxially with the first axis, the guide member having a tubular part for locating in a bore hole of a template for guiding the working path of the handpiece during use and a flange which extends perpendicularly with respect to the first axis for limiting the depth of the rotary dental tool within the bore hole.

The provision of a tubular guide member on the dental handpiece has an advantage of allowing accurate control of the position of a tool, as the tool is inserted into a surgical template. As the guide member is mounted to the handpiece, there is no need to remove bushings from a surgical template after drilling operations using the handpiece.

The handpiece may also include a drive for rotating the retained tool about the first axis. The drive may be a motorised drive such as a pneumatic, hydraulic or electrical drive or components for a manual drive.

A further aspect of the present invention is that the housing has an inlet through which a tool can be inserted and the handpiece comprises a mechanism for retaining the guide member which is positioned at the inlet. The guide member can be attached to, or attachable to, a mounting part which is mountable around the housing. These features can provide the advantage that assembly of the various parts for mounting the tool are located close to each other and are therefore easy to assemble.

Preferably, the guide member is positionable at a varying distance from the mounting part. This has the advantage that the tool which is mountable on the handpiece can be adjusted in its position.

Preferably, the guide member is removably mountable to the handpiece. This provides the advantage that a variety of guide members may be provided and also cleaning of the handpiece is made easier. For example, a set of guide members can be provided. Further described is that guide members can differ in the dimensions of at least one of: flange depth in the direction along the first axis and radius in a direction perpendicular to the first axis. This allows a user to quickly select, and fit, a guide member which has dimensions matched to the particular bore hole diameter and depth of an implant site.

Further described is that the dental handpiece may include or be adapted to operate with a display which is arranged to display an identification of which guide member from the set of guide members should be fitted to the handpiece. This allows the operator to confirm easily that the correct guide member has been used.

The mounting of the guide member can be adapted such that it is movable, in use, along the first axis. This has an advantage that the guide member provides guidance at an early stage of using the tool, to help ensure that the tool is correctly centred with a bore hole, while also allowing the guide member to retract as the tool (e.g. drill) penetrates a work piece.

Further described is that the guide member can be biased into a position in which it extends from the housing, the guide member being movable to reduce the amount of protrusion as the handpiece engages with a work piece.

Another feature is the provision of a channel within the housing and the guide member is retractable within the channel.

Further described is that the guide member can comprise a set of tubes which are arranged to telescope. This is an additional way to allow adjustment of the guide member and its operation.

A further aspect of the present invention is that the adjustment mechanism which adjusts the position of a retained tool with respect to the housing, in the direction of the first axis, to vary the length of tool which projects from the housing. This allows optimum positioning of the tool. This adjustment may be manual, motorised or automatic.

To provide additional accuracy, a control for manual adjustment of the position of the tool can be provided. For example, the adjustment mechanism can be arranged to act on the tool retaining mechanism to move the tool retaining mechanism along the first axis.

In a further aspect the drive comprises a geared transmission having a first transmission component and a second transmission component, and wherein the adjustment mechanism is operable to vary the position of the first transmission component with respect to the second transmission component in the direction of the first axis. This is a convenient way to allow adjustment along the first axis.

A further aspect is that the dental handpiece may include or be adapted to operate with a display which is arranged to display a setting which the adjustment mechanism should be set to, for a surgical operation. The dental handpiece may also include or be adapted to operate with a display which is arranged to display a distance by which a tool is required to protrude from the housing. A display allows the operator to confirm that the correct setting has been achieved in each case.

A further aspect is that means may be provided to automatically set a distance by which a tool is required to protrude from the housing or may confirm that a distance set by a dental operative or set otherwise is the correct one.

### BRIEF DESCRIPTION OF THE DRAWINGS

Embodiments of the invention will be described, by way of example only, with reference to the accompanying drawings in which:
Figure 1 shows a conventional swan-necked dental handpiece;
Figure 2 shows a cross-section of a scan prosthesis used during a preparatory stage of an implant operation;
Figure 3 shows a drilling jig (surgical template) used when drilling holes in the jawbone of a patient and fitting dental implants;
Figure 4 shows a first embodiment of a handpiece according to the present invention with a guide member fitted around an inlet to the head of the handpiece;
Figures 5a and 5b show how guide members can be interchangeably fitted to the head of the handpiece;
Figures 6a and 6b show another embodiment of a handpiece according to the present invention having a guide member which is retractable within the head;
Figure 7 shows apparatus used to install a dental implant and the dimensions of the apparatus;
Figures 8a-8c show how difference in tool length can be compensated according to the present invention;
Figure 9 shows a mechanism used within the head of the handpiece for clamping a shank of a tool;
Figure 10 shows a typical tool in the form of a drill which can be retained by the clamping mechanism of Figure 9;
Figure 11 shows a first embodiment of a mechanism for varying the position of the clamping mechanism within the head;
Figure 12 shows another embodiment of a mechanism for varying the position of the clamping mechanism within the head;
Figure 13 shows a guide member which fits around a head of a handpiece;
Figures 14a and 14b show a guide member which fits around a head of a handpiece and which can be positioned at varying distances from the head;
Figure 15 shows how a set of guide parts can be interchangeably fitted to the guide member of Figures 14a and 14b.

### DESCRIPTION OF PREFERRED EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

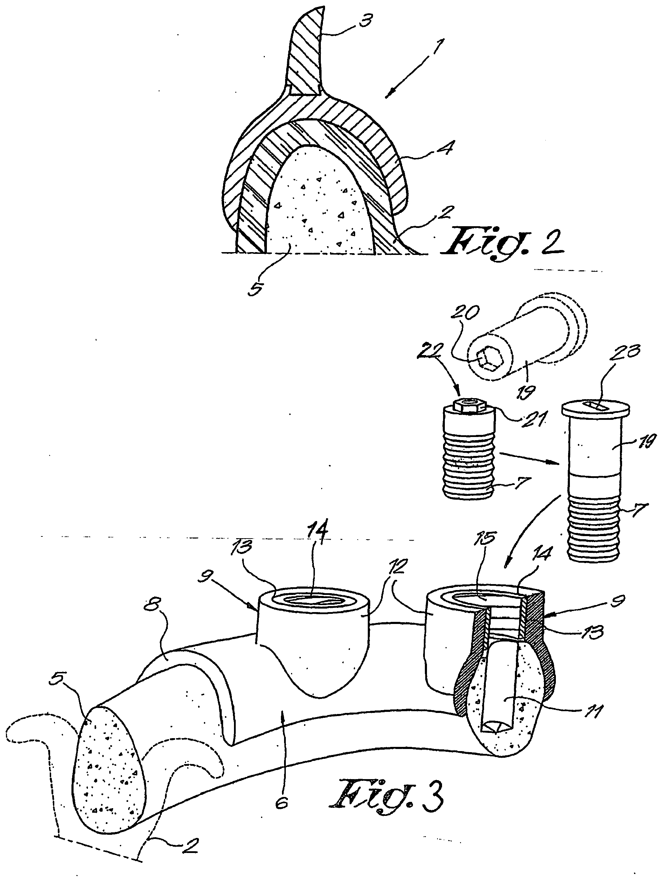

Before describing the drilling assembly in detail, the initial steps of an implant process and the apparatus used in the process will be described. The aim of the process is to create a dental superstructure which will fit on implants which have been fixed in the jawbone of a patient. The superstructure supports artificial teeth. With the intention of creating a superstructure, a diagnostic setup of the future teeth will first be made. This is normally achieved using an articulator, i.e. an appliance in which two teeth molds or plaster models/casts can be positioned in correct relation to one another enabling the simulation of realistic jaw movement. The diagnostic setup is made on plaster models of the remaining teeth or gums that indicates the future positions of the teeth. The same test arrangement is also copied in a radio-opaque material in order to make a scan prosthesis 1, as represented in Figure 2, the purpose of which will become clear from the further description. According to a variant of the method, instead of realizing this diagnostic setup in a mechanical articulator, the test arrangement can also be made virtually, with a computer, by means of what is called a virtual articulator which can simulate the movements of the upper jaw in relation to the lower jaw. In this case, the jaws of the patient or a cast thereof will be scanned, for example with a laser scanner. The two scanned jaws are positioned in relation to one another by registering the respective teeth surfaces on each other, or by scanning one of the jaws with a mouldable paste on top of it, such that the surface of one jaw corresponds exactly to the other jaw.

Next, teeth can be chosen from a digital library and positioned in those places where teeth are missing. After this preliminary stage, preferably as a first step of the actual method, a computer planning is made in view of the placement of the implants. This can be done, for example, by first scanning the patient with a computed tomography scanner (CT-scanner) and by simulating the implants on the CT-scans, as described in the Belgian patent No. 1.011. 205. It is useful that the patient is scanned with what is called a scan prosthesis 1 as represented in Figure 2. This is a copy of the loose prosthesis of the patient or of the diagnostic setup made by means of an articulator. This scan prosthesis 1, which is placed on the gums or mucosa 2 during the scanning, is made of a radio-opaque material which is thus visible in the CT-images, whereby the teeth 3 of this scan prosthesis 1 have another degree of opacity than the base part 4 supported on the gums or the mucosa 2, which covers the bone of the patient.

The teeth 3 can be made of any suitable material such as acrylic resin mixed with 30% of barium sulfate, while the rest of the prosthesis 1 is made of a material with a different radio opacity or other property compared to the material for the teeth so that the teeth can be distinguished from the prosthesis. For example, the rest of the prosthesis 1 can be made from a mixture of acrylic resin and 10% of barium sulfate. This offers the advantage that the teeth 3 are nicely visible in the CT scan images and can thus be segmented separately in a simple manner. In addition, the shape of the gums 2 will also be visible, as the base part 4 of the scan prosthesis 1 can be identified and delineates the gums and its lower side represents the shape of the gums. Furthermore, the shape of the surface of the bone 5 is perfectly visible by means of the CT-scan. Next, a drill jig or surgical template 6 is created. This drill jig can possibly also serve as a fitting jig, since the implants 7 can be placed by means of it. Implant placement can possibly also be achieved with a separate fitting jig. The template 6, and possibly the fitting jig, can for example be made by means of Rapid Prototyping techniques, as described in the Belgian patent No. 1.011. 205.

According to Figure 3, the template 6 fits on part 8 of the bone 5 of the patient (after the gums 2 have been opened) and enables pre-operative transferal of the drill directions in conformity with the planning of the surgeon. To this end, the template 6 has ducts 9 for one or several drills 10. It should be noted that the bone 5 may have a very irregular surface. As the template 6 is designed based on data coming from the CT-scan, the part of the jig in contact with the bone will have an inner surface 8 which always follows the shape of the irregular surface very precisely. The result is that there will always be an accurate positioning. It should also be noted that, according to a variant, said template, fitting jig and possibly even said positioning jig, can be provided with a contact part which is not or not solely designed to be supported on the bone 5, but (also) cooperates with parts of the gums 2 and/or remaining teeth of the patient The template 6 is used when drilling holes 11 for implants 7. The template 6 has been designed such that it can be used for all implants 7. This template 6 is put only once on the patient and is possibly screwed down temporarily. The ducts 9 are, as shown, preferably composed of several parts. First, there are a number of guiding tubes 12, preferably in the form of collars, which make up one piece together with the contact part of the template 8. The ducts 9 are lined with optional bore tubes 14 which are made of, for example, metal, or could be lined with a metal coating or sleeve. The bore tubes 14 may or may not be present in the template. If no bore tubes 14 are present, the tubular guide members (35 - mentioned below in the description of the handpiece) mounted on the handpiece will engage directly in the guiding tubes 12 of the template. Alternatively, they will engage in the metal bore tubes 14.

If the template 6 is to be used as a fitting jig, implants are fitted through the bore tubes 14. Implants 7 are individually mounted to holders 19.

Figure 4a schematically shows the head of a handpiece according to an embodiment of the present invention. The handpiece can be used in connection with the surgical template 6 as part of an implant process. The principal components of the head include a clamping mechanism 60 (referred to as "C" in Fig. 1) for holding the shank 28 of a tool (referred to as "E" in Fig. 1), such as a drill, bur or implant driver. A driving mechanism 31, 32 (referred to as "D" in Fig. 1) transmits torque to the shank 28 to rotate the tool. Typically, the driving mechanism 31, 32 is driven by a drive shaft or belt (not shown) which connects to a remote motor or takes the form of a pneumatic turbine which is driven by a pneumatic supply line. Alternatively, the head can include an electric motor which receives an electrical supply via a connecting cable. Any suitable drive can be used, e.g. pneumatic, hydraulic or electrical. An annular cover plate 33 (referred to as "B" in Fig. 1) fits across the lower face of the head and provides an inlet through which the shank 28 of the tool can be inserted. The cover plate 33 provides a hygienic seal between the inner components of the handpiece head and the surrounding environment. Tools can be fitted to, and removed from, the head as required via a quick-change mechanism. The components are held within a housing 27 (referred to as "A" in Fig. 1). The features described above are well known in conventional handpieces and do not require further explanation.

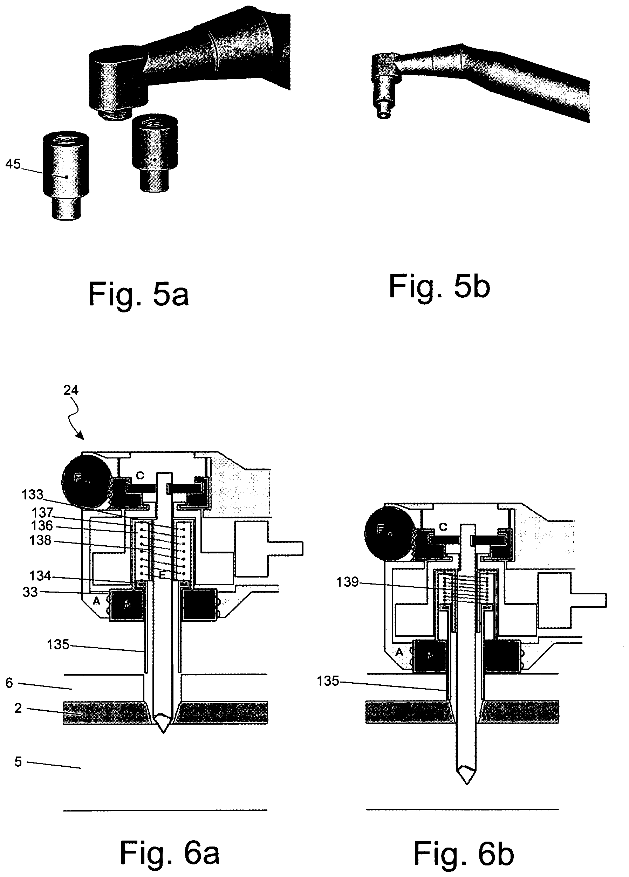

A component 35 connects to the cover plate 33 and serves, in use, as a guide member which can guide (centre) a tool within a bore hole in a template 6. Guide member 35 is a tubular part, which optionally may have a flange 37 at the upper end, i.e. proximal to the head 24. The flange 37 serves as a stop which prevents the tool driven by the handpiece from being inserted beyond a defined depth into the bore hole in a template. It is preferred (but not required) that the guide member 35 is removably connectable to the handpiece, as shown in Figures 5a and 5b. This allows other guides (e.g. of different dimensions) to be fitted and allows cleaning. For example, the inner surface of the guide member 35 includes fixings 36 which cooperate with complementary fixings on the stem of the cover plate 33 to secure the guide member 35 in place. The fixings 36 can include any suitable form of fixing, e.g. a screw thread, a button-operated quick-release mechanism, bayonet connection, magnet, etc. The guide member 35 will be described more fully below.

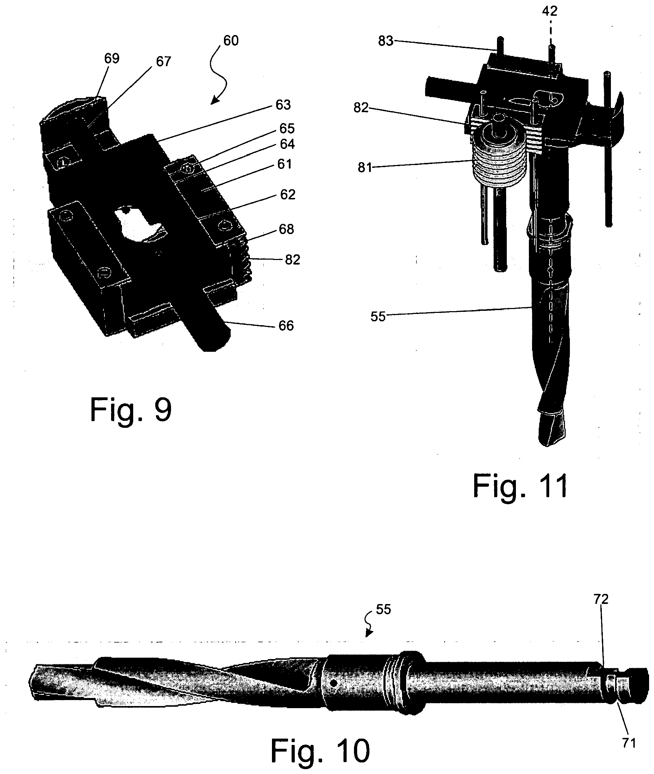

The handpiece also includes an adjustment mechanism 40, 41 which can modify and/or set and/or display and/or confirm the length of the tool protruding from the handpiece. In various embodiments of the present invention, the adjustment mechanism includes a manually adjustable or an electronic control actuator 40 which moves clamping mechanism 60 along axis 42. To illustrate this, Figure 4b shows the same apparatus with the shank 28 of the tool retracted further inside the head. Another adjustment mechanism is shown more fully in Figure 11. The electronic control actuator can be adapted to set a distance that a tool protrudes, e.g. in accordance with an output of a planning software. The electronic control actuator 40 can be driven to the required position. The manually adjustable or electronic control actuator 40 may also be adapted to measure or record a distance related to the protrusion distance. Electronic control circuits may then confirm whether the final protrusion distance is the correct distance as input by the dental operative or as output from a computer planning. The electronic control circuits may be located on the dental handpiece or they may be located remotely therefrom but in communication therewith, e.g. via a cable or a wireless connection.

The mechanism to adjust or to display or to set or to confirm the length of the tool protruding from the head of the handpiece may be implemented in a variety of ways using any suitable type of actuator, e.g. a mechanical (as shown in Figs. 4 and 11), an electrical, a pneumatic, a hydraulic or a magnetic actuator. For example, the adjustment mechanism can be magnetic. For instance, the clamping mechanism 60 may be joined to a magnet which is surrounded by a coil to form a solenoid linear actuator. When an electrical current is applied to the coil, the magnet and thus the clamping mechanism will move relative to the coil, which is fixed in the housing of the handpiece. The magnitude of the electrical current may be used to control the extent of the displacement.

In Figure 4a the guide member 35 is removably fitted to the cover plate 33, around the inlet to the head. Figure 6a shows an alternative embodiment of the invention in which a guide member 135 is retractable into the head, along an annular channel 136. The channel 136 is defined by an outer tubular wall 137, an inner tubular wall 138 and an annular end face 133. A resilient member such as a spring 139 is held captive in the channel between a flange 134 at the upper end of guide member 135 and the end face 133 of the channel. Figure 6a shows the guide member in the most extended position, with the spring 139 fully extended to press flange 134 against the cover plate 33. Figure 6b shows the same apparatus with the guide member 135 retracted partway along channel 136, against the bias of the resilient member, e.g. spring 139. The guide member can be moved in this manner when it contacts soft tissue 2 at the base of a bore hole. When the handpiece is removed from the bore hole, resilient member, e.g. spring 139 exerts a restoring force which forces guide member 135 to return to the position shown in Figure 6a.

It is noted that the guide member 135 does not need to extend over the entire length of the tool. In order to overcome the problem of delayed guidance of the tool the guide member 135 retracts into the head of the handpiece. The advantage of this feature comes from the fact that tool guidance can be provided in an earlier stage of the tool manipulation. Because the guide member can retract inside the housing, a longer guide member and therefore a guide which is more useful during the early stages of drilling can be provided compared to a fixed guide which fits to the cover plate. The guide member can be replaceable in this embodiment but requires loosening of the cover plate 33.

A retractable guide can be used in the same way as a guide member which mounts to the cover plate. For example, it can be used to vary the depth of drilling. Once the resilient member, e.g. spring 139 has been compressed the allowed vertical translation is blocked. If a flange is added on the guide member, the drill depth can also be limited.

The dimensions (inner and outer diameter, height) of the guide member 35, 135 are chosen as a function of the implant in a manner which will now be described. Features such as the implant brand, product line, connection type, shoulder width, etc. will determine the dimensions of the guide member. Typical dental implant treatment requires the placement of multiple implants. In the case of guided implantology, where a surgical template is used to prepare the implant cavities and to guide the implant placement, ergonomic considerations may dictate that only one template is used for both the drilling and for the placing of all implants. From an ease-of-use point of view it is preferable that all implants within a same implant line, regardless of their lengths, could be installed using the same tools.

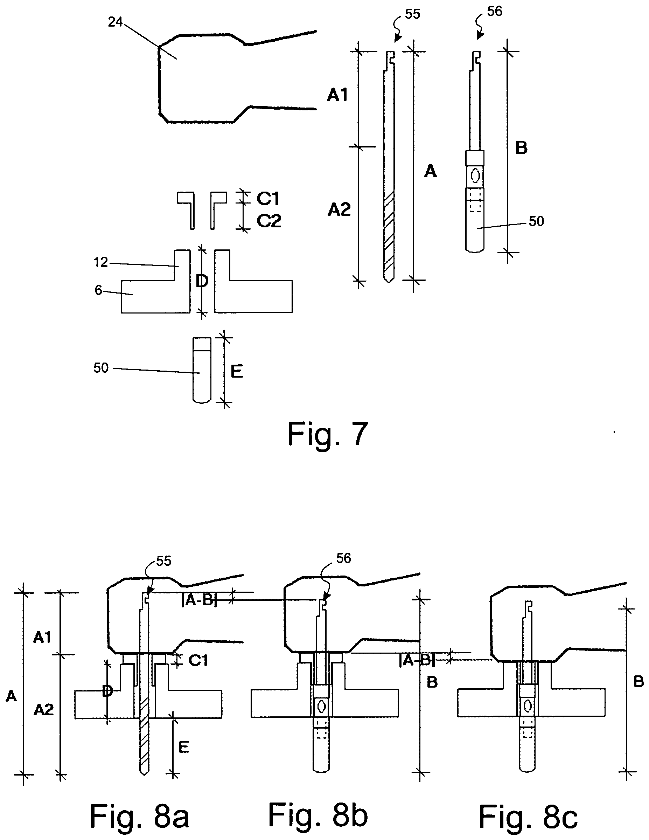

Consider the illustrative hypothetical case of a fully edentulous patient. A surgical template is manufactured which fits exactly on the jaw of the patient. The template is provided with a number of bore tubes that indicate the entry point and the inclination of the implants according to an implant plan created in a planning software such as SimPlant™. The implants need to be installed level with the crest of the bone. Referring to Figure 7, a first implant 50 has a length E. The depth of the bore tube 12 corresponding to this implant in the surgical template 6 is D. To prepare the implant cavity a dedicated set of drills is available from the implant manufacturer. An example drill 55 is shown. The drills have varying diameters and a fixed length A. A drill engages in the handpiece over a distance A1 with the remaining length A2 protruding from the head of the handpiece. A centering component is mounted on the head of the handpiece and is inserted into the bore tube 12 over a distance C2. The component has a flange with a thickness C1 that acts as a physical stop. In order to prepare an implant cavity with a correct depth for the given example, the distance A2 must be equal to the sum of the distances C1, D and E. To achieve this one, or a combination, of different variables can be modified:
(i) the depth (D) of the bore tube 12;
(ii) the thickness (CI) of the flange of the guide member;
(iii) the length (A1) of the tool engaged in the handpiece.

In the case of the retractable guide, as illustrated in figures 6a and 6b, the flange thickness C1 equals zero, i.e. no flange has been provided on the guide member. This means that the cover plate itself acts as a physical stop limiting the depth of the tool penetration.

The depth D of the bore tube 12 is determined in accordance to a given implant plan. It can be specified during the design of the surgical template, since the device is custom made per patient. However, it cannot be modified during surgery.

The thickness of the flange C1 is fixed per component. Nevertheless, a set of centering components can be provided with varying thicknesses of the respective flanges. The components can be mounted on the handpiece in a removable manner. In this manner, the guide member 35 and optionally 135 if provided with a flange performs the functions of centering/guiding a tool in a bore tube and of ensuring that a drill creates a hole of a required depth. With respect to the retractable guide member 135, to obtain the same effect, one must take into account the extent of retractability of the guiding member 135 in the head of the handpiece and position the flange with the appropriate thickness C1 at the correct position along its circumference.

The mechanism 40, 41 described above in the head of the handpiece can be used to vary the length (A1) of the tool engaged in the handpiece. This offers the advantage of flexibility. The mechanism 40, 41 can be used alone, or in combination with guide members 35 of different dimensions. The mechanism 40, 41 has an advantage that no additional components are needed and changes can be made per-operatively.

In the above mentioned example the preparation of the implant cavity will be followed by the implant placement. Typically, implant manufacturers supply a tool 56 known as an implant driver for this purpose. The total length of this tool, with an implant 50 attached to the tool, is B. Installing the implant 50 at the correct depth is recommended if the part of the tool protruding from the handpiece A2 = C1 + D + E. Typically, this is not the case. Therefore, either a different centering component must be mounted (e.g. a centring component with a different flange thickness C1) or the position of the clamping mechanism must be changed. Relatively, the distance that needs to be compensated between using tools 55 and 56 equals A-B.

In figure 8b distance A-B is compensated by changing the position of the clamping mechanism inside the handpiece, either manually, in a motorised way or automatically thereby changing the length of the implant driver-implant assembly protruding from the head.

Figure 8c shows an alternative way of compensating for distance A-B. Relative to figure 8a the position on the tool in the head of the handpiece remains unaltered. However, the thickness of the flange C1 is changed (from A-B to zero). The effect relative to the depth of the implant placement in the bone is identical.

According to the above hypothetical example, the next implant to be installed has a length F which is slightly longer length than the first implant, where F = E+e. In order for a surgeon to use the same tools, compensating for the difference in implant length is required. Thus, in comparison to the first implant either the depth of the bore tube is decreased with a value e. This can be achieved by fitting another centering component having a reduced flange thickness of (C1 - e) or the position of the clamping mechanism is modified by a distance e along the direction of the movement of the tool (axis 42, Figure 4a). This modification can be done manually, in a motorised way or automatically.

Thus, the present invention provides full control over the installation of the implants. Modifying the variables C1 and A1 allows the user to compensate for variations in implant length, e.g. compensating manually, in a motorised way or automatically. In addition, they can also be used to compensate for the presence of soft tissue underlying the surgical template, which influences the penetration depth required to accurately install the implants according to a given implant plan.

According to the present invention the values for variables D, C1 and A1 can be calculated from the implant planning. The information can be transferred to the surgical setting by means of a custom information sheet detailing which values to use for the installation of which implant. The information may also be transferred by a communication path, e.g. a communication link by cable or wireless. This transfer may be automatic. Any other method of transfer may be used, e.g. the information may be stored on a memory device such as a USB memory stick, and this memory device plugged into the dental handpiece allowing transfer of the data.

A number of other measures can be taken to make the system more user-friendly. Firstly, the guide members can be colour-coded as a function of their length and/or diameter. Figures 5a and 5b show colour-coded identification marks 45.

Figure 5a shows the handpiece and two different guiding members, e.g. the left one could be blue, and the right one could be grey. The thickness of the flange (CI) is different for the left (blue) and the right (grey) member. Figure 5b shows the left (blue) guiding member after mounting on the cover plate of the handpiece.

Alternatively, the guide members can have a specific code marked on them, such as by engraving. This coding helps to identify the correct components during the transfer of the planning to apparatus required for the operating theatre. A digital or analogue readout may be provided on the handpiece indicating how value A1 (or a derived measure) changes when operating the mechanism to change the position of the clamping. Alternatively some means of mechanical calibration could be provided.
For example, instead of having a manually operated wheel to modify the position of the clamping mechanism inside the head of the handpiece, the change in position could be driven separately using a small electronic motor. An electronic readout, e.g. a form of position encoder, coupled to the movement of the motor would display how the position of the clamping mechanism changes. Given that the planning is known beforehand, the required positions of the clamping mechanism could be programmed into the rotary tool or could be provided to the rotary tool as indicated above, e.g. via a communication link. The setting of the position of the clamping mechanism could be done automatically, e.g. in function of an output of a computer planning done previously. Any other method of transfer may be used, e.g. the programming information may be stored on a memory device such as a USB memory stick, and this memory device plugged into the dental handpiece allowing transfer of the data.
The electronic display could then show which implant is being installed and what the required length is. Also the display could confirm that the correct implant is being installed at the correct depth for instance.

As a further measure to aid usage, markings can be made on the surgical template 6 to indicate which implant (type, length, diameter, reference number etc.) should be installed per bore tube.

The handpiece has a handle that is coaxial with the rotation axis of a drive motor, followed by the neck at an angle of 15° to 30° to the handle and a head that is perpendicular or substantially perpendicular to the neck and into which is fit a rotary tool (drill, bur, implant driver, trephine, etc.) with a cylindrical shank that is gripped in a clamping mechanism. For insertion or removal of the tool a variety of mechanisms are possible. Such mechanisms may for example use push buttons or pressure piston, actuation levers, wedge shape plungers or other actuation means to disengage the clamping mechanism retaining the tool. Figure 9 shows an example of a possible clamping device. Two components 61, 63, each having a respective slot 62, are movable relative to one another along a glide bearing 68. A spring 67 acts between a wall 69 and an end wall of component 63 and serves to bias component 63 into the rest position shown in which the openings 62, 64 are aligned such that a rim 65 engages with the end 71 of a tool 55 (shown in Figure 10) to retain the tool within the head. A push button 66, which protrudes through the housing of the handpiece head, can be used to compress the spring 67 and thereby alter the relative positions of the components 61, 63, thereby allowing the tool 55 to be inserted or removed. Components 61, 63 thus cooperate as jaws to enlarge and constrict the opening.
Indeed, the purpose of this clamping mechanism is to hold the tool in a vertical position. It does not hinder the tool in rotation around its axis. The drive 32 engages with the tool by acting on a notch 72 at the top of the tool as best shown in Figure 10.

Referring again to Figure 4a, the head of the handpiece further has a member 32 for rotating the shank of the tool. Member 32 engages with a driving part 31 which forms part of the neck. The member 32 can be of the crankpin type, or a pinion or a friction wheel. Alternatively, a magnetic field may be used to induce rotation of member 32 (e.g. using the same principle as an electromotor). In the present configuration both the clamping mechanism and the member transmitting the movement to the tool can be set at different positions along axis 42. This setting may be done manually, in a motorised way or automatically. By doing so the length of the tool protruding from the handpiece is modified. For example, according to one solution, shown in Figure 11, the positions of components 61, 63, transmission member 32 and the tool 55 are varied by turning a spiral thumb wheel 81 that meshes with teeth 82 provided on component 61. Component 61 is guided by one or more rails which are aligned parallel with the axis 42. As wheel 81 is adjusted, component 61 changes position along axis 42, carrying with it tool 55 and the transmission member 32. The wheels in figures 4 and 11 are different. This illustrates further that that several alternatives exist for moving the clamping mechanism 60

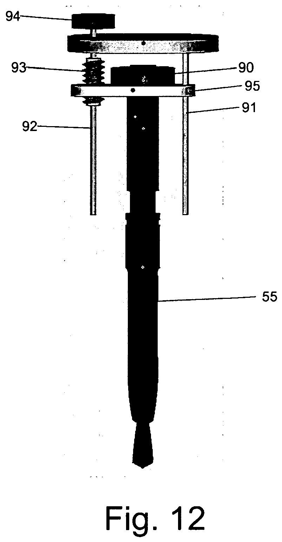

Figure 12 shows a further alternative embodiment of the adjusting mechanism. As before, a tool 55 is clamped by a clamping mechanism 90 which is the same as previously shown in Figure 9. The clamping mechanism 90 is supported by a plate 95 which is constrained to follow guide rails 91, 92. Guide rail 92 has a screw member 93 which cooperates with a screw-threaded hole in the plate 95. A manual control knob 94 is fitted to the upper end of rail 92. Rail 92 protrudes through the housing 27 of the head and knob 94 is mounted outside of the housing. The position of the tool can be modified by turning knob 94.

Although mechanical control knobs have been described above the present invention includes any other way of providing the movement including the use of an actuator, e.g. to motorise the movement and/or to automate it.

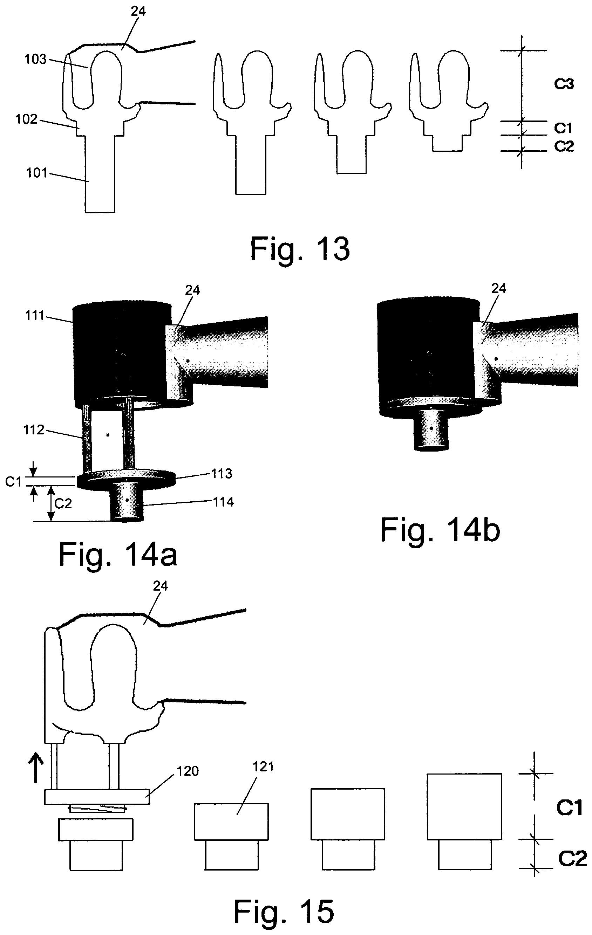

The guide member engages in acrylic or metal bore tubes (20) provided in a surgical template (21) that fits exactly on a specified region of the patient's anatomy. The dimensions of the guide member are dictated by the implant brand and type used. The diameters of the guide member, for example, will be adapted such that brand specific tools such as drills, taps and implant drivers fit inside. Thus the tools will be centred inside the bore tubes of the template and guided during use. In addition, the component may be provided with a physical stop, for instance in the shape of a flange or a collar, that is blocked against the surgical template 6, limiting the penetration depth of the tool in the surgical template and thereby also in the underlying tissues. Alternatively such a physical stop may be provided on the cover plate or directly on the housing of the handpiece. For example, as an alternative, a special cover plate of the housing may be designed which, due to it's design, can fulfil the function of a physical stop for depth control. For instance, the cover plate may have a cone like shape, with its base engaging with the housing of the handpiece and the top having a flat surface, the diameter of which corresponds to the diameter of the guiding tube in the template. When the cover plate hits the guiding tube further penetration into the bone is blocked. In the embodiment shown in Figures 4a and 4b a guide member which fits to the cover plate at the inlet to the head. Figures 13-15 show a further embodiment of a guide member which is provided with a cap which fits to the head of the handpiece. This has an advantage that no modification is required to the cover plate to secure the guide member. The cap can locate in fixings which are specially provided on the head, for the purpose of receiving and securing the cap. Alternatively, the cap can be arranged to provide all of the functions of securing the cap to the head without any modifications to the head itself. This allows the cap to be provided as an after market accessory for users of existing heads. The cap can be retained by means of a strap, by means of a combination of the shape of the cap (partially embracing the head) and the resilience of the material from which the cap is formed.

The guide member comprises several parts which can be provided either in assembly or in one piece: (i) a guiding part 101 having a length C₂ which engages in the bore tube of a surgical template, (ii) a collar/flange 102 having a thickness C1, which acts as depth control and (iii) a mounting part 103 for attachment to the handpiece. The mounting part 103 can use a screw fitting, a bayonet fitting, a snap-on interface, or any other suitable form or, as explained above, can simply snap around the head. Per implant, a set of components with an identical collar height is chosen. The collar height determines the depth to which a tool will be driven into a patient. During the tool manipulation components with subsequently shorter guiding parts are used until finally the depth stop is reached. Guidance is thus guaranteed over the entire length of the tool.

If no retractable guide is used a problem can be caused by the fact that guidance is limited to the upper part of the rotary tool. To overcome this problem, a set of guiding members can be used. During use, the depth of the implant cavities will gradually increase with each new guiding member used until finally the desired depth is reached. For example, the number of members can be limited to two or three guiding members.

The guide member can provide depth control and tool guidance. A set of guide members can be provided which have differently-dimensioned collars 102 and/or guide parts 101. Alternatively, a single mounting part 103 and a set of differently-dimensioned guide and collar parts 101, 102 can be provided as a kit, with the appropriate guide and collar combination 101, 102 being fitted to the mounting part 103. Figures 14a and 14b show a further alternative of the guide member in which a collar 113 and guide part 114 is connected to a mounting part 111 by a set of rails 112. The collar and guide start in the extended position as shown in Fig.14a (to offer maximum guidance) and then retract to what is shown in Fig.14b. This is another embodiment of the retractable guiding member as shown in figure 6a and 6b. However, the retraction system is now incorporated in a dedicated component that is mounted on/over the head of the handpiece allowing it to be provided as a solution for existing handpieces.

Several components with different collar thickness C1 are required to provide depth control.

According to a variation of the abovementioned component, the mounting part of the component that incorporates the retraction mechanism is fitted with a base plate 120. To this base plate, parts 121 can be attached -for instance by means of a screw thread - with different collar heights C1 (for depth control) and a guiding length C2. In this way components with different collar thickness C1 can be provided.

According to yet another variation, depth control is provided by a feature engaging directly with the retraction mechanism that limits the extent to which either the guiding part or the base plate can retract. For example, this could be similar to the arrangement shown in Fig.14 or Fig.15 but with the collar and guide parts fixed at a particular distance from the mounting part.. A way of achieving this is by having a component (say a clip) which engages over the guiding bearings of the retraction system and thus limits the possible retraction. The clip would be positioned in between the base plate and the mounting part.

According to another embodiment of the invention the handpiece may also be in the shape of a manually powered device such as a wrench that transmits the torque to the tool. The centering component can be mounted on the wrench to provide control over the depth of for instance the implant placement. Also a positioning mechanism may be provided in the head of the wrench to move the tool along a direction parallel to its longitudinal axis. The wrench can be a purely manual tool with no electrical/pneumatic drive.

The present invention is hence not be limited to swan-like handpieces, but includes within its scope manual driving tools and handpieces with alternative designs (straight handpieces being the most obvious example). The invention is not limited to the embodiments described herein, which may be modified or varied without departing from the scope of the invention.

## Claims

1. A dental handpiece comprising: a housing (27) having an inlet through which a shank (28) of a rotary dental tool (28, 55) can be inserted; a tool retaining mechanism (60, 90) for releasably retaining the shank of the rotary dental tool, the mechanism retaining the tool such that the tool extends from the housing, through the inlet, along a first axis (42); and an adjustment mechanism (40, 41, 81, 82, 93, 94, 95) arranged to act on the tool retaining mechanism (60, 90) to adjust the position of the tool retaining mechanism within the housing, in the direction of the first axis (42), to thereby vary the length of tool which projects from the housing.

2. A dental handpiece according to claim 1, wherein adjustment mechanism can be any of a manual, motorised or automatic adjustment mechanism.

3. A dental handpiece according to claim 1, further comprising a drive (32) for rotating a retained tool about the first axis.

4. A dental handpiece according to claim 3 wherein the drive (32) comprises a geared transmission having a first transmission component and a second transmission component, and wherein the adjustment mechanism (40, 41, 81, 82, 93, 94, 95) is operable to vary the position of the first transmission component (31) with respect to the second transmission component (32) in the direction of the first axis.

5. A dental handpiece according to any one of claims 1 to 4 wherein the adjustment mechanism is continuously variable over a range of values.

6. A dental handpiece according to any one of claims 1 to 5 further comprising a display which is arranged to display a setting which the adjustment mechanism should be set to for a surgical operation.

7. A dental handpiece according to any one of claims 1 to 6 further comprising a display which is arranged to display a distance by which a tool is required to protrude from the housing.

8. A dental handpiece according to any one of claims 1 to 7 further comprising means for automatically setting a distance by which a tool is required to protrude from the housing.

9. A dental handpiece according to any one of claims 1 to 8, further comprising means to confirm the setting of a distance by which a tool is required to protrude from the housing.

10. A dental handpiece according to claim 8, wherein the means for automatically setting the distance is adapted to set the distance in function of an output of a computer planning.

11. A dental handpiece according to any one of claims 1 to 10 further comprising a tubular guide member (35) mounted coaxially with the first axis, the guide member having a tubular part for locating in a bore hole of a template for guiding the working path of the handpiece during use and a flange which extends perpendicularly with respect to the first axis for limiting the depth of the rotary dental tool within the bore hole.

12. A dental apparatus comprising a dental handpiece according to claim 11 and a template having at least one bore hole which defines a position at which the rotary dental tool is to be used, the bore hole having a diameter which is substantially equal to an outer diameter of the guide member.

13. A method of adjusting a dental handpiece having a housing (27) with an inlet through which a shank (28) of a rotary dental tool (28, 55) can be inserted; a tool retaining mechanism for releasably retaining the shank of the rotary dental tool, the mechanism retaining the tool such that the tool extends from the housing, through the inlet, along a first axis; the method comprising: a
cting on the tool retaining mechanism (60, 90) to adjust the position of the tool retaining mechanism within the housing, in the direction of the first axis, (42) to thereby vary the length of tool which projects from the housing.

14. The method according to claim 13, further comprising automatically setting a distance by which a tool is required to protrude from the housing.

15. The method according to claim 14, wherein the setting of the distance by which a tool is required to protrude from the housing comprises setting the distance in function of an output of a computer planning.

## Patentansprüche

1. Dentalhandstück, das Folgendes umfasst: ein Gehäuse (27), das einen Einlass aufweist, durch den ein Schaft (28) eines sich drehenden Dentalwerkzeugs (28, 55) eingesetzt werden kann, einen Werkzeug-Festhaltemechanismus (60, 90) zum lösbaren Festhalten des Schafts des sich drehenden Dentalwerkzeugs, wobei der Mechanismus das Werkzeug derart festhält, dass sich das Werkzeug von dem Gehäuse aus, durch den Einlass, entlang einer ersten Achse (42) erstreckt,
und einen Einstellmechanismus (40, 41, 81, 82, 93, 94, 95), der dafür angeordnet ist, auf den Werkzeug-Festhaltemechanismus (60, 90) einzuwirken, um die Position des Werkzeug-Festhaltemechanismus innerhalb des Gehäuses, in der Richtung der ersten Achse (42), einzustellen, um dadurch die Länge des Werkzeugs, die von dem Gehäuse aus vorspringt, zu verändern.

2. Dentalhandstück nach Anspruch 1, wobei der Einstellmechanismus ein beliebiger von einem manuellen, einem motorisierten oder einem automatischen Einstellmechanismus sein kann.

3. Dentalhandstück nach Anspruch 1, das ferner einen Antrieb (32) zu Drehen eines festgehaltenen Werkzeugs um die erste Achse umfasst.

4. Dentalhandstück nach Anspruch 3, wobei der Antrieb (32) ein Zahnradgetriebe umfasst, das einen ersten Getriebebestandteil und einen zweiten Getriebebestandteil aufweist, und wobei der Einstellmechanismus (40, 41, 81, 82, 93, 94, 95) funktionsfähig ist, um die Position des ersten Getriebebestandteils (31) in Bezug auf den zweiten Getriebebestandteil (32) in der Richtung der ersten Achse zu verändern.

5. Dentalhandstück nach einem der Ansprüche 1 bis 4, wobei der Einstellmechanismus über einen Bereich von Werten kontinuierlich veränderlich ist.

6. Dentalhandstück nach einem der Ansprüche 1 bis 5, das ferner eine Anzeige umfasst, die dafür angeordnet ist, eine Einstellung anzuzeigen, auf die der Einstellmechanismus für eine chirurgische Operation eingestellt werden sollte.

7. Dentalhandstück nach einem der Ansprüche 1 bis 6, das ferner eine Anzeige umfasst, die dafür angeordnet ist, eine Entfernung anzuzeigen, um die ein Werkzeug von dem Gehäuse aus vorspringen muss.

8. Dentalhandstück nach einem der Ansprüche 1 bis 7, das ferner Mittel zum automatischen Einstellen einer Entfernung, um die ein Werkzeug von dem Gehäuse aus vorspringen muss, umfasst.

9. Dentalhandstück nach einem der Ansprüche 1 bis 8, das ferner Mittel zum Bestätigen der Einstellung einer Entfernung, um die ein Werkzeug von dem Gehäuse aus vorspringen muss, umfasst.

10. Dentalhandstück nach Anspruch 8, wobei die Mittel zum automatischen Einstellen der Entfernung dafür eingerichtet sind, die Entfernung in Abhängigkeit von einer Ausgabe einer Rechnerplanung einzustellen.

11. Dentalhandstück nach einem der Ansprüche 1 bis 10, das ferner ein röhrenförmiges Führungselement (35) umfasst, das koaxial mit der ersten Achse angebracht ist, wobei das Führungselement einen röhrenförmigen Teil zum Positionieren in einem Bohrloch einer Schablone zum Führen des Arbeitsweges des Handstücks während der Verwendung und einen Flansch, der sich senkrecht in Bezug auf die erste Achse erstreckt, zum Begrenzen der Tiefe des sich drehenden Dentalwerkzeugs innerhalb des Bohrlochs aufweist.

12. Dentalvorrichtung, die ein Dentalhandstück nach Anspruch 11 und eine Schablone, die wenigstens ein Bohrloch aufweist, das eine Position definiert, an der das sich drehende Dentalwerkzeug verwendet werden soll, umfasst, wobei das Bohrloch einen Durchmesser aufweist, der im Wesentlichen gleich einem Außendurchmesser des Führungselements ist.

13. Verfahren zum Einstellen eines Dentalhandstücks, das ein Gehäuse (27) mit einem Einlass, durch den ein Schaft (28) eines sich drehenden Dentalwerkzeugs (28, 55) eingesetzt werden kann, einen Werkzeug-Festhaltemechanismus zum lösbaren Festhalten des Schafts des sich drehenden Dentalwerkzeugs, wobei der Mechanismus das Werkzeug derart festhält, dass sich das Werkzeug von dem Gehäuse aus, durch den Einlass, entlang einer ersten Achse (42) erstreckt, aufweist, wobei das Verfahren Folgendes umfasst:
Einwirken auf den Werkzeug-Festhaltemechanismus (60, 90), um die Position des Werkzeug-Festhaltemechanismus innerhalb des Gehäuses, in der Richtung der ersten Achse (42), einzustellen, um dadurch die Länge des Werkzeugs, die von dem Gehäuse aus vorspringt, zu verändern.

14. Verfahren nach Anspruch 13, das ferner das automatische Einstellen einer Entfernung, um die ein Werkzeug von dem Gehäuse aus vorspringen muss, umfasst.

15. Verfahren nach Anspruch 14, wobei das Einstellen der Entfernung, um die ein Werkzeug von dem Gehäuse aus vorspringen muss, das Einstellen der Entfernung in Abhängigkeit von einer Ausgabe einer Rechnerplanung umfasst.

## Revendications

1. Pièce à main dentaire comprenant : un logement (27) ayant une entrée à travers laquelle une tige (28) d'un outil dentaire rotatif (28, 55) peut être insérée ; un mécanisme de retenue d'outil (60, 90) pour retenir de façon amovible la tige de l'outil dentaire rotatif, le mécanisme retenant l'outil de sorte que l'outil s'étend à partir du logement, à travers l'entrée, le long d'un premier axe (42) ; et un mécanisme d'ajustement (40, 41, 81, 82, 93, 94, 95) agencé pour agir sur le mécanisme de retenue d'outil (60, 90) pour ajuster la position du mécanisme de retenue d'outil à l'intérieur du logement, dans la direction du premier axe (42), pour faire varier de ce fait la longueur d'outil qui dépasse du logement.

2. Pièce à main dentaire selon la revendication 1, dans laquelle le mécanisme d'ajustement peut être n'importe lequel d'un mécanisme d'ajustement manuel, motorisé ou automatique.

3. Pièce à main dentaire selon la revendication 1, comprenant en outre un entraînement (32) pour faire tourner un outil retenu autour du premier axe.

4. Pièce à main dentaire selon la revendication 3, dans laquelle l'entraînement (32) comprend une transmission par engrenages ayant un premier composant de transmission et un second composant de transmission, et dans laquelle le mécanisme d'ajustement (40, 41, 81, 82, 93, 94, 95) est opérationnel pour faire varier la position du premier composant de transmission (31) par rapport au second composant de transmission (32) dans la direction du premier axe.

5. Pièce à main dentaire selon l'une quelconque des revendications 1 à 4, dans laquelle le mécanisme d'ajustement est variable de façon continue sur une plage de valeurs.

6. Pièce à main dentaire selon l'une quelconque des revendications 1 à 5, comprenant en outre un afficheur qui est agencé pour afficher un réglage auquel le mécanisme d'ajustement devrait être mis pour une opération chirurgicale.

7. Pièce à main dentaire selon l'une quelconque des revendications 1 à 6, comprenant en outre un afficheur qui est agencé pour afficher une distance de laquelle on exige qu'un outil dépasse du logement.

8. Pièce à main dentaire selon l'une quelconque des revendications 1 à 7, comprenant en outre un moyen pour régler automatiquement une distance de laquelle on exige qu'un outil dépasse du logement.

9. Pièce à main dentaire selon l'une quelconque des revendications 1 à 8, comprenant en outre un moyen pour confirmer le réglage d'une distance de laquelle on exige qu'un outil dépasse du logement.

10. Pièce à main dentaire selon la revendication 8, dans laquelle le moyen pour automatiquement régler la distance est conçu pour régler la distance en fonction d'une sortie d'une planification informatique.

11. Pièce à main dentaire selon l'une quelconque des revendications 1 à 10, comprenant en outre un élément de guidage tubulaire (35) monté de façon coaxiale avec le premier axe, l'élément de guidage ayant une partie tubulaire pour le placement dans un trou de forage d'un modèle pour guider le chemin de travail de l'instrument pendant l'utilisation et une bride qui s'étend perpendiculairement par rapport au premier axe pour limiter la profondeur de l'outil dentaire rotatif à l'intérieur du trou de forage.

12. Appareil dentaire comprenant une pièce à main dentaire selon la revendication 11 et un modèle ayant au moins un trou de forage qui définit une position à laquelle l'outil dentaire rotatif doit être utilisé, le trou de forage ayant un diamètre qui est sensiblement égal à un diamètre extérieur de l'élément de guidage.

13. Procédé d'ajustement d'une pièce à main dentaire ayant un logement (27) avec une entrée à travers laquelle une tige (28) d'un outil dentaire rotatif (28, 55) peut être insérée ; un mécanisme de retenue d'outil pour retenir de façon amovible la tige de l'outil dentaire rotatif, le mécanisme retenant l'outil de sorte que l'outil s'étend à partir du logement, à travers l'entrée, le long d'un premier axe ; le procédé comprenant :
l'action sur le mécanisme de retenue d'outil (60, 90) pour ajuster la position du mécanisme de retenue d'outil à l'intérieur du logement, dans la direction du premier axe, (42) pour faire varier de ce fait la longueur d'outil qui dépasse du logement.

14. Procédé selon la revendication 13, comprenant en outre le réglage automatique d'une distance de laquelle on exige qu'un outil dépasse du logement.

15. Procédé selon la revendication 14, dans lequel le réglage de la distance de laquelle on exige qu'un outil dépasse du logement comprend le réglage de la distance en fonction d'une sortie d'une planification informatique.
